# EUROPEAN PATENT SPECIFICATION

(11) **EP 3 978 921 B1**
(45) Date of publication and mention of the grant of the patent: **04.03.2026**
(21) Application number: 20813991.5
(22) Date of filing: 21.05.2020
(51) Int. Cl.: G01N 33/48, G01N 33/53, G01N 33/78

(54) **METHOD AND REAGENT FOR MEASURING THYROGLOBULIN**
VERFAHREN UND SYSTEM ZUR MESSUNG VON THYREOGLOBULIN
MÉTHODE ET RÉACTIF POUR LA MESURE DE LA THYROGLOBULINE

(30) Priority: 24.05.2019 JP 2019097525
(43) Date of publication of application: 06.04.2022
(73) Proprietor: Fujirebio Inc., Tokyo 107-0052 (JP)
(72) Inventor: OHUE, Chiharu, Hachioji-shi, Tokyo 192-0031 (JP); YAGI, Shintaro, Hachioji-shi, Tokyo 192-0031 (JP); AOYAGI, Katsumi, Hachioji-shi, Tokyo 192-0031 (JP)
(74) Representative: Mewburn Ellis LLP
(86) International application number: PCT/JP2020/020104
(87) International publication number: WO 2020/241443

(56) References cited:
- WO-A1-2014/122973
- WO-A1-2018/047792
- JP-A- 2000 241 429
- JP-A- 2007 010 418
- JP-A- 2017 032 583
- JP-A- H08 297 123
- CHAN C. ET AL: "Human autoantibodies to thyroglobulin are directed towards a restricted number of human specific epitopes", CLINICAL AND EXPERIMENTAL IMMUNOLOGY, 1 September 1987 (1987-09-01), Oxford, pages 516 - 523, XP093033981, Retrieved from the Internet <URL:https://www.ncbi.nlm.nih.gov/pmc/articles/PMC1542372/pdf/clinexpimmunol00108-0034.pdf> [retrieved on 20230322]
- NISHIKAWA, MITSUSHIGE : "Thyroglobulin (Tg)", THE JAPANESE JOURNAL OF CLINICAL MEDICINE, vol. 68, no. 7, 2010, pages 295 - 298, XP009513914, ISSN: 0047-1852

## Description

### TECHNICAL FIELD

The present invention relates to a measurement method and a measurement reagent for thyroglobulin.

### BACKGROUND ART

Thyroglobulin (Tg) is a glycoprotein having a molecular weight of 660,000 produced only in thyroid follicular cells. The biosynthesized Tg is released into the follicular lumen. In this process, binding of iodine molecules to tyrosine groups in the Tg molecule occurs by the action of peroxidase, to cause synthesis of thyroid hormone. The Tg in the follicular lumen is ingested again by follicular cells, and digested in the follicular cells, causing release of thyroid hormone. This process is activated by the action of thyroid-stimulating hormone (TSH). Thus, under normal conditions, release of Tg itself into blood hardly occurs, and release of Tg into blood indicates a certain abnormality of the thyroid. Therefore, Tg is an extremely useful marker for thyroid diseases because of its high organ specificity. In particular, Tg in blood is used as a marker for evaluation of operations for differentiated thyroid cancer, and as a marker for knowing the presence or absence of postoperative recurrence or metastasis. Further, Tg in blood is, for example, useful as an index of a therapeutic effect on, or remission of, Basedow's disease, and also useful for determination or differentiation of the disease type, or for therapeutic monitoring, of congenital hypothyroidism. It has also been suggested that combination of blood Tg with diagnostic imaging may enable preoperative diagnosis of nodular goiter, and differential diagnosis between a benign thyroid disease and a malignant tumor.

However, in cases where the subject is positive for anti-thyroglobulin antibody (TgAb), a low Tg value may be found due to a problem in the measurement even when the actual value of Tg is high. For example, since 20 to 30% of patients with thyroid cancer are positive for TgAb, simultaneous measurement of TgAb has been necessary for measurement of Tg. Further, accurate measurement of the Tg level has been difficult in cases of Hashimoto's disease with TgAb positivity, and similarly, there has been a concern that the Tg level measured in cases of another autoimmune disease exhibiting TgAb positivity (Basedow's disease) may be inaccurate.

In order to solve this problem, the present applicants previously invented, and filed a patent application for, a method of treating a sample with a pretreatment liquid containing one or both of a surfactant and an acidifier (Patent Document 1). Although the method according to Patent Document 1 is effective, the antibody used in the immunoassay needs to be resistant to the acidifier and/or the surfactant.

### PRIOR ART DOCUMENT

### [Patent Document]

[Patent Document 1] WO 2018/047792

### SUMMARY OF THE INVENTION

### PROBLEMS TO BE SOLVED BY THE INVENTION

If the Tg level can be measured accurately even for the TgAb-positive patients described above, the Tg level may be widely applicable to therapeutic monitoring of thyroid diseases. An object of the present invention is to provide a measurement method and a measurement reagent for thyroglobulin, which measurement method and measurement reagent enable more accurate measurement of the thyroglobulin level by a single test without being influenced by interference of anti-thyroglobulin antibody, and which measurement method and measurement reagent allow use of even an antibody less resistant to an acidifier and/or a surfactant in an immunoassay.

### MEANS FOR SOLVING THE PROBLEMS

In order to achieve the above object, the present inventors intensively studied to discover that, in measurement of thyroglobulin in a biological sample, a more accurate thyroglobulin measurement value can be obtained without being influenced by anti-thyroglobulin antibody, by carrying out, before subjecting the biological sample to immune reaction, a pretreatment step of mixing the biological sample with a pretreatment liquid containing an alkaline substance, thereby completing the present invention.

The present invention provides the following.
(1) A method of measuring, by an immunoassay, thyroglobulin in a sample separated from a living body, the method comprising a pretreatment step of mixing the sample separated from a living body with a pretreatment liquid containing an alkaline substance, wherein the pretreatment liquid further contains at least one selected from the group consisting of nonionic surfactants, zwitterionic surfactants, anionic surfactants, and urea.
(2) The method according to (1), wherein the final concentration of the alkaline substance in the pretreatment step is more than 0.05 N and not more than 0.5 N.
(3) Use of an alkaline substance for pretreatment in an immunoassay of thyroglobulin, wherein a pretreatment liquid for the pretreatment further contains at least one selected from the group consisting of nonionic surfactants, zwitterionic surfactants, anionic surfactants, and urea.

### EFFECT OF THE INVENTION

The present invention can provide a measurement method and a measurement reagent for thyroglobulin (Tg), which method and reagent allow, even in a biological sample containing anti-thyroglobulin antibody (TgAb), release of Tg from TgAb to reduce the influence of their interaction, thereby enabling more accurate measurement of the amount of Tg contained in the sample. Further, the measurement method and the measurement reagent enable use of an antibody less resistant to an acidifier and/or a surfactant in an immunoassay.

### MODE FOR CARRYING OUT THE INVENTION

Unless otherwise specified, each "%"-based concentration described in the present description represents a weight/volume (w/v, g/100 mL)-based concentration.

### <Method of Measuring Thyroglobulin>

The thyroglobulin (Tg) to be measured by the present invention is Tg derived from an arbitrary animal. The Tg is preferably Tg derived from a mammal (for example, a primate such as a human, a monkey, or a chimpanzee; a rodent such as a mouse, a rat, or a rabbit; a pet animal such as a dog or a cat; a domestic animal such as a pig or a cow; or a working animal such as a horse or a sheep), more preferably Tg derived from a primate, especially preferably Tg derived from a human.

### 1. Pretreatment Step

The method of the present invention is a method in which Tg present in a biological sample is measured using immune reaction by reacting the biological sample with an antibody. The method is characterized in that it includes a pretreatment step of mixing the biological sample with a pretreatment liquid before the immune reaction (reaction step). By the pretreatment step, Tg can be brought into a state in which it is released from autoantibody (TgAb) or the like. The pretreatment liquid contains an alkaline substance.

The volume ratio between the biological sample and the pretreatment liquid to be mixed in the pretreatment step is preferably 1:10 to 10:1, more preferably 1:5 to 5:1, still more preferably 1:3 to 3:1. The biological sample to be used in the present invention is not limited as long as it is a sample that may contain Tg, and examples of the biological sample include serum, plasma, whole blood, urine, stool, oral mucosa, pharyngeal mucosa, intestinal mucosa, and biopsy specimens (for example, thyroid fine needle aspiration cytology (fine needle aspiration: FNA) specimens, intestinal specimens, and liver specimens). The biological sample is preferably serum or plasma.

Preferred examples of the alkaline substance contained in the pretreatment liquid include: alkali metal hydroxides such as sodium hydroxide and potassium hydroxide; and alkaline earth metal hydroxides such as magnesium hydroxide. The normality of the alkaline substance in the pretreatment liquid, in terms of the final concentration during the pretreatment, is preferably more than 0.05 N and not more than 0.5 N, especially preferably not less than 0.1N and not more than 0.4N. In cases where the normality of the alkaline substance is more than 0.05 N and not more than 0.5 N, the effect of the pretreatment can be sufficiently obtained, and influence on the subsequent reaction step can be minimized.

In the present invention, the pH after the pretreatment liquid containing the alkaline substance is mixed with the sample is, for example, not less than pH 10.0, preferably not less than pH 11.0, more preferably not less than pH 12.0 although the pH may vary depending on the alkaline substance added. Further, in the present invention, the pH after the pretreatment liquid containing the alkaline substance is mixed with the sample is, for example, not more than pH 13.7, preferably not more than pH 13.5, more preferably not more than pH 13.3 although the pH may vary depending on the alkaline substance added. More specifically, the pH after the pretreatment liquid containing the alkaline substance is mixed with the sample is, for example, pH 10.0 to 13.7, preferably pH 11.0 to 13.5, more preferably pH 12.0 to 13.3. In cases where the pH in the pretreatment step is within these ranges, the effect of the pretreatment can be sufficiently obtained, and influence on the subsequent reaction step can be minimized.

The pretreatment liquid further contains at least one selected from the group consisting of nonionic surfactants, zwitterionic surfactants, anionic surfactants, and urea. By this, the sensitivity of the immunoassay can be improved. Examples of the nonionic surfactants include polyoxyethylene alkylphenyl ether (trade name, Triton X-100 or the like) and polyoxyethylene alkyl ether (trade name, Brij 35 or the like). Examples of the zwitterionic surfactants include 3-[(3-cholamidopropyl)dimethylammonio]propanesulfonate (CHAPS). *N*-dodecyl-*N*,*N-*dimethyl-3-ammonio-1-propanesulfonate (C12APS), *N*-tetradecyl-*N*,*N*- dimethyl-3-ammonio-1-propanesulfonate (C14APS), and *N*-hexadecyl-*N*,*N*-dimethyl-3-ammonio-1-propanesulfonate (C16APS). Examples of the anionic surfactants include sodium dodecyl sulfate (SDS), sodium *N*-lauroyl sarcosine (NLS). lithium dodecyl sulfate, sodium dodecylbenzene sulfonate, and deoxycholic acid.

In cases where the pretreatment liquid contains the surfactant or urea, the concentration of the nonionic surfactant in terms of the final concentration during the pretreatment is preferably 0.01% to 5%, more preferably 0.05% to 5% from the viewpoint of improvement of the sensitivity. The final concentration of the zwitterionic surfactant is preferably 0.01% to 1%. The final concentration of the anionic surfactant is preferably 0.01% to 2.5%. The final concentration of urea is preferably 0.01 to 0.1 M.

When necessary, the pretreatment liquid may contain another protein denaturant such as thiourea. The concentration of the denaturant, in terms of the concentration during the treatment, is preferably not less than 0.1 M. more preferably not less than 0.5 M and less than 4 M. For enhancement of the effect of the treatment, the pretreatment liquid may contain any of monosaccharides, disaccharides, citric acid, and citric acid salts, or a combination of these. The pretreatment liquid may also contain a chelating agent such as EDTA.

The pretreatment step may be carried out by simply mixing the biological sample with the pretreatment liquid, and leaving the resulting mixed liquid to stand at room temperature. Although the mixed liquid may be heated (at a temperature of, for example, 35°C to 95°C), the pretreatment step is preferably carried out at room temperature from the viewpoint of simplicity and cost. The pretreatment time is preferably not less than 1 minute, more preferably not less than 3 minutes, still more preferably not less than 5 minutes. Although there is no upper limit of the pretreatment time, the pretreatment time may be not more than 30 minutes, especially not more than 15 minutes. After the pretreatment, the alkaline substance is preferably neutralized with an acid such as hydrochloric acid.

### 2. Reaction Step

The mixed liquid of the biological sample obtained by the pretreatment step in the method of the present invention is subsequently subjected to the reaction step of immunoassay. In the reaction step, the mixed liquid of the biological sample is mixed with a buffer, and antigen in the mixed liquid is reacted with an antibody against Tg. Regarding the immunoassay itself of Tg, a variety of methods are well known, and any immunoassay capable of quantification of Tg may be employed.

Examples of the buffer include those based on MES buffer, phosphate buffer, Tris buffer, or carbonate buffer. Buffers based on phosphate buffer may be especially preferably used. In cases where a pretreatment liquid containing a surfactant is used, it is preferred, for the purpose of absorbing unreacted surfactant, to use a buffer containing a water-soluble polymer such as BSA, polyvinyl pyrrolidone (PVP), polyvinyl alcohol (PVA), or dextran sulfate sodium at about 0.01 to 10.0%, especially 0.05 to 5.0% in terms of the final concentration after mixing with the pretreated mixed liquid. The mixed liquid of the pretreatment step and the buffer are mixed together at a volume ratio of preferably 1:10 to 10:1, more preferably 1:5 to 5:1, still more preferably 1:3 to 3:1.

The antibody against Tg to be used in the method of the present invention is an antibody that recognizes at least part of the amino acid sequence of Tg as an epitope. The antibody against Tg is not limited, and any antibody that recognizes a known epitope may be used. The antibody against Tg is preferably an antibody that recognizes a Tg-specific epitope (especially a human Tg-specific epitope).

The antibody against Tg may be either a polyclonal antibody or a monoclonal antibody. The antibody against Tg may be any isotype of immunoglobulin (for example, IgG, IgM, IgA, IgD, IgE, or IgY). The antibody against Tg may be a full-length antibody. The full-length antibody means an antibody containing a heavy chain and a light chain each having a variable region and a constant region (for example, an antibody containing two Fab portions and Fc portions). The antibody against Tg may also be an antibody fragment derived from such a full-length antibody. The antibody fragment is part of a full-length antibody, and examples of the antibody fragment include antibodies lacking the constant region (for example, F(ab')₂, Fab', Fab, or Fv). The antibody against Tg may also be a modified antibody such as a single-chain antibody.

The antibody against Tg may be prepared using a conventionally known method. For example, the antibody against Tg may be prepared using the above-described epitope as an antigen. Alternatively. since a number of antibodies against Tg that recognize the above-described epitopes are commercially available, such commercially available products may also be used.

The antibody against Tg may be immobilized on a solid phase. In the present description, an antibody immobilized on a solid phase may be simply referred to as an immobilized antibody. Examples of the solid phase include solid phases in/on which a liquid phase can be stored or loaded (for example, supports such as plates, membranes, and test tubes; and containers such as well plates, microchannels, glass capillaries, nanopillars, and monolith columns) and solid phases that can be suspended or dispersed in a liquid phase (for example, solid-phase carriers such as particles). Examples of the material of the solid phase include glasses, plastics, metals, and carbons. As the material of the solid phase, a non-magnetic material or a magnetic material may be used. From the viewpoint of simplicity of operation and the like, the material is preferably a magnetic material. The solid phase is preferably a solid-phase carrier, more preferably a magnetic solid-phase carrier, still more preferably a magnetic particle. As the method for immobilization of the antibody, a conventionally known method may be used. Examples of such a method include physical adsorption, covalent bonding, use of an affinity substance (biotin, streptavidin, or the like), and ionic bonding. In a particular embodiment, the antibody against Tg is an antibody immobilized on a solid phase, preferably an antibody immobilized on a magnetic solid phase, more preferably an antibody immobilized on a magnetic particle.

In the reaction step, after the mixing of the mixed liquid of the pretreatment step with the buffer, the resulting mixture may be brought into contact with the immobilized antibody. Alternatively. for example, an antibody immobilized on particles may be preliminarily included in a buffer to provide a particle liquid, followed by mixing the above mixed liquid with the particle liquid. Although the reaction step may be carried out by a primary reaction step alone as in the immunoagglutination method or the competitive method, a secondary reaction step may also be provided as in the sandwich method. In cases where the secondary reaction step is provided, a washing step for removal of an unreacted component(s) may be provided between the primary reaction step and the secondary reaction step.

The antibody against Tg may be labeled with a labeling substance. In the present description, an antibody labeled with a labeling substance may be simply referred to as a labeled antibody. Examples of the labeling substance include enzymes (for example, peroxidase, alkaline phosphatase, luciferase, β-galactosidase), affinity substances (for example, streptavidin, biotin), fluorescent substances and proteins (for example, fluorescein, fluorescein isothiocyanate, rhodamine, green fluorescent protein, red fluorescent protein), luminescent or light-absorbing substances (for example. luciferin, aequorin, acridinium, ruthenium), and radioactive substances (for example, ³H, ¹⁴C, ³²P, ³⁵S, ¹²⁵I). In cases where the secondary reaction is provided in the method of the present invention, the antibody to be used for the secondary reaction may be labeled with such a labeling substance.

In a particular embodiment, the method of the present invention includes another antibody against Tg as the antibody to be used for the secondary reaction, which antibody recognizes an epitope different from that of the antibody against Tg used for the primary reaction. Details of such an epitope recognized by the other antibody are the same as the details of the epitope of the above-described antibody against Tg (however, in the case of combined use, the epitopes are different). The combination of the epitope recognized by the antibody against Tg and the epitope recognized by the other antibody against Tg is not limited. Use of such another antibody is preferred in cases where, for example, the sandwich method is used.

### 3. Detection Step

The method of the present invention may further comprise a step of detecting binding of the antibody against Tg to the target antigen (Tg) in the reaction step. In cases where a label is used for the antibody used for the primary reaction or secondary reaction in the reaction step, the detection is possible by a method suitable for the label used. For example, in cases where an enzyme label is used, the detection is possible by adding a substrate of the enzyme. For example, in cases where alkaline phosphatase (ALP) is used for the labeled antibody, 3-(2'-spiroadamantane)-4-methoxy-4-(3'-phosphoryloxy)phenyl-1,2-dioxetane disodium salt (AMPPD) may be used as the enzyme substrate to provide a system of the chemiluminescent enzyme immunoassay (CLEIA) method.

The method of the present invention is an immunoassay using an antibody against Tg. Examples of such an immunoassay include the direct competitive method, indirect competitive method, and sandwich method. Examples of such an immunoassay include chemiluminescent enzyme immunoassay (CLEIA), chemiluminescence immunoassay (CLIA), turbidimetric immunoassay (TIA), enzyme immunoassay (EIA) (for example, direct competitive ELISA, indirect competitive ELISA, and sandwich ELISA), radioimmunoassay (RIA), latex agglutination, fluoroimmunoassay (FTA), and immunochromatography. These immunoassays *per se* are well known, and do not need to be described herein in detail. A brief description, however, of each immunoassay is given below.

The direct competitive method is a method in which an antibody against a target antigen to be measured (in the present invention, Tg) is immobilized on a solid phase (the solid phase and the immobilization are as described above), and blocking treatment (treatment of the solid phase with a solution of a protein such as serum albumin) for prevention of non-specific adsorption is carried out, followed by reacting this antibody with a test sample containing the target antigen (in the present invention, a biological sample subjected to the pretreatment step as described above) and a certain amount of labeled antigen (the label is as described above), performing washing, and then quantifying the label bound to the solid phase. Since the antigen in the test sample and the labeled antigen competitively bind to the antibody, as the amount of the antigen in the test sample increases, the amount of the label bound to the solid phase decreases. Antigen standard solutions with various known concentrations are prepared, and the amount of the label (the absorbance, luminescence intensity, fluorescence intensity, or the like depending on the properties of the label; the same applies hereinafter) immobilized on the solid phase is measured for each solution. Thereafter, a calibration curve is prepared such that the antigen concentration and the amount of the label are plotted on the abscissa and the ordinate, respectively. By measuring the amount of the label for an unknown test sample, and applying the measured amount of the label to the calibration curve, the amount of the antigen in the unknown test sample can be measured. The direct competitive method *per se* is well known in the art, and described in, for example, US 20150166678 A.

In the indirect competitive method, a target antigen (in the present invention, Tg) is immobilized on a solid phase (the solid phase and the immobilization are as described above). Subsequently, blocking treatment of the solid phase is carried out and then a test sample containing the target antigen (in the present invention, a biological sample subjected to the pretreatment step as described above) is mixed with a certain amount of an anti-target-antigen antibody, followed by reacting the resulting mixture with the immobilized antigen. After washing, the anti-target-antigen antibody bound to the solid phase is quantified. This can be carried out by reacting a labeled secondary antibody (the label is as described above) against the anti-target-antigen antibody, performing washing, and then measuring the amount of the label. Antigen standard solutions with various known concentrations are prepared, and the amount of the label immobilized on the solid phase is measured for each solution to prepare a calibration curve. By measuring the amount of the label for an unknown test sample, and applying the measured amount of the label to the calibration curve, the amount of the antigen in the unknown test sample can be measured. It is also possible to use a labeled primary antibody without using the labeled secondary antibody. The indirect competitive method *per se* is well known in the art, and described in, for example, the above-mentioned US 20150166678 A.

Among the sandwich methods, the forward sandwich method is a method in which an anti-target-antigen antibody is immobilized on a solid phase (the solid phase and the immobilization are as described above), and blocking treatment is carried out, followed by reaction with a test sample containing a target antigen (in the present invention, a biological sample subjected to the pretreatment step as described above), washing, reaction with a labeled secondary antibody against the target antigen (the label is as described above), washing, and then quantification of the label bound to the solid phase. Among the sandwich methods, the reverse sandwich method is a method in which a secondary antibody is preliminarily reacted with a test sample, and an antigen-antibody complex produced by binding between the secondary antibody and the target antigen is reacted with an immobilized antibody (primary antibody), followed by washing and then quantification of the label bound to the solid phase. There is also a sandwich method in which an immobilized antibody, a test sample, and a secondary antibody are reacted at the same time. Antigen standard solutions with various known concentrations are prepared, and the amount of the label immobilized on the solid phase is measured for each solution to prepare a calibration curve. By measuring the amount of the label for an unknown test sample, and applying the measured amount of the label to the calibration curve, the amount of the antigen in the unknown test sample can be measured. The sandwich method *per se* is well known in the art, and described in, for example, US 20150309016 A.

Among the immunoassays described above, chemiluminescent enzyme immunoassay (CLEIA), chemiluminescence immunoassay (CLIA), enzyme immunoassay (EIA), radioimmunoassay (RIA), and fluoroimmunoassay (FIA) are immunoassays classified based on the type of the label to be used when the direct competitive method, indirect competitive method, sandwich method, or the like described above is carried out. Chemiluminescent enzyme immunoassay (CLEIA) is an immunoassay which uses an enzyme (for example, the above-described alkaline phosphatase) as the label, and a substrate (for example, the above-described AMPPD) that generates a chemiluminescent compound as the substrate. Enzyme immunoassay (EIA) is an immunoassay which uses an enzyme (for example, the above-described peroxidase, alkaline phosphatase, luciferase, β-galactosidase or the like) as the label. As the substrate of each enzyme, a compound quantifiable by measurement of the absorbance or the like is used. For example, in cases of peroxidase, 1,2-phenylenediamine (OPD), 3,3'5,5'-tetramethylbenzidine (TMB), or the like is used. In cases of alkaline phosphatase, *p*-nitrophenyl phosphate (pNPP) or the like is used. In cases of β-galactosidase, MG: 4-methylumbelliferyl galactoside, NG: nitrophenyl galactoside. or the like is used. In cases of luciferase, luciferin or the like is used. Radioimmunoassay (RIA) is a method which uses a radioactive substance as a label. Examples of the radioactive substance include radioactive elements such as ³H, ¹⁴C, ³²P, ³⁵S, and ¹²⁵I as described above. Fluoroimmunoassay (FIA) is a method which uses a fluorescent substance or a fluorescent protein as the label. Examples of the fluorescent substance or the fluorescent protein include, as described above, fluorescein, fluorescein isothiocyanate, rhodamine, green fluorescent protein, red fluorescent protein and the like. Immunoassays *per se* using these labels are well known in the art, and described in, for example, US 8039223 B and US 20150309016 A.

Turbidimetric immunoassay (TIA) is an immunoassay which utilizes the phenomenon that an antigen-antibody complex produced by antigen-antibody reaction between a target antigen to be measured (in the present invention, Tg) and an antibody against this antigen causes an increase in the turbidity. The antigen is added, at various known concentrations, to an anti-target-antigen antibody solution, and the turbidity of each resulting mixture is measured to prepare a calibration curve. By similarly measuring the turbidity of an unknown test sample, and applying the measured turbidity to the calibration curve, the amount of the antigen in the unknown test sample can be measured. Turbidimetric immunoassay *per se* is well known in the art, and described in, for example, US 20140186238 A. Latex agglutination is a method similar to turbidimetric immunoassay, but uses a suspension of latex particles whose surfaces have an anti-target-antigen antibody immobilized thereon, instead of the antibody solution in turbidimetric immunoassay. Turbidimetric immunoassay and latex agglutination *per se* are well known in the art, and described in, for example, US 7820398 B.

Immunochromatography is a method in which the above-described sandwich method or competitive method is carried out on a substrate (also called a matrix or a strip) formed with a porous material such as filter paper, cellulose membrane, glass fiber, or non-woven fabric. For example, in cases of immunochromatography by the sandwich method, a detection zone on which an anti-target-antigen antibody is immobilized is provided on the substrate, and a test sample containing a target antigen (in the present invention, a biological sample subjected to the pretreatment step as described above) is added to the substrate, followed by allowing a developer to flow from the upstream side. This allows the target antigen to migrate to the detection zone, where the target antigen is immobilized on the detection zone. The immobilized target antigen is sandwiched with a labeled secondary antibody, and the label immobilized on the detection zone is detected to detect the target antigen in the test sample. By forming a label zone containing the labeled secondary antibody in the upstream side of the detection zone, the complex of the target antigen and the labeled secondary antibody can be immobilized on the detection zone. In cases where the label is an enzyme, a substrate zone containing a substrate of the enzyme is also provided in the upstream side of the detection zone. In cases of the competitive method, for example, the target antigen may be immobilized on the detection zone, and the target antigen in the test sample may be allowed to compete with the target antigen immobilized on the detection zone. By providing a labeled antibody zone in the upstream side of the detection zone, allowing the target antigen in the test sample to react with the labeled antibody, immobilizing unreacted labeled antibody on the detection zone, and then detecting or quantifying the label, the target antigen in the test sample can be detected or quantified. Immunochromatography *per se* is well known in the art, and described in, for example, US 6210898 B.

### <Measurement Reagent for Tg>

The measurement reagent for Tg of the present invention is a measurement reagent that can realize the above-described measurement method for Tg. The measurement reagent of the present invention is characterized in that it contains an alkaline substance in addition to the constitution used for ordinary immunoassays.

The reagent of the present invention contains the constituting components in a form in which they are isolated from each other, or in the form of a composition. More specifically, the constituting components may be provided in a form in which they are stored in different containers (for example, tubes or plates), or some of the constituting components may be provided in the form of a composition (for example. in a single solution). Alternatively, the reagent of the present invention may be provided in the form of a device. More specifically, the reagent may be provided in a form in which all constituting components are stored in a device. Alternatively, the reagent may be provided in a form in which some of the constituting components are stored in a device while the other constituting components are not stored in the device (for example, in a form in which the other constituting components are stored in a separate container(s)). In such a case, the constituting components not stored in the device may be used by injection into the device when measuring the target substance.

In a preferred embodiment, the reagent of the present invention may have a constitution suitable for the type of the immunoassay to be employed. For example, in cases where the sandwich method is employed, the reagent of the present invention may contain, as indispensable constituting components, i) a pretreatment liquid, ii) an antibody against Tg, and iii) a buffer; and, as arbitrary constituting components, iv) another antibody against Tg, v) a labeling substance, vi) a diluent, and, when necessary, vii) a substrate that reacts with the labeling substance. The constituting components ii) and iii) may be contained in a single solution. The constituting component iv) may be labeled with the labeling substance v). The antibody against Tg may be preferably immobilized on a magnetic particle.

### EXAMPLES

### <Example 1 Test for Confirmation of Effect of Alkalization Pretreatment>

### (1) Preparation of Anti-Thyroglobulin Antibody Plate

To a polystyrene 96-well microwell plate (Thermo F16 Black Maxisorp), an antibody dilution (0.1 M sodium hydrogen carbonate, 0.1 M sodium chloride; pH 9.6) containing 2 µg/mLmouse anti-human Tg antibody 64-1 (manufactured by Fujirebio Inc.) was dispensed at 100 µL/well, and the plate was then incubated at 4°C overnight. The microwell plate was washed with PBS three times, and then a blocking liquid (1.0% BSA, 3% sucrose, PBS) was dispensed at 350 µL/well, followed by incubation at room temperature for 3 hours. After removing the blocking liquid, the plate was dried under vacuum to provide an anti-Tg antibody plate.

### (2) Preparation of Alkaline Phosphatase-Labeled Mouse Anti-Human Tg Monoclonal Antibody

Mouse anti-human Tg antibody 5E6 (manufactured by AbD Serotec) was enzymatically labeled according to a conventional method, to prepare an alkaline phosphatase-labeled mouse anti-human Tg monoclonal antibody (labeled antibody). The labeled antibody was diluted with an antibody diluent (0.02 M KH₂PO₄, 0.076 M K₂PO₄, 0.25 M NaCl, 1% PVP, 1% BSA, 0.05% casein Na, 0.05% Tween 20 (trade name; pH 7.0)) to 0.5 µg/mL, to prepare a labeled antibody liquid.

### (3) Sample Preparation

To purchased serum (Tg concentration, 10 ng/mL) of a healthy individual, human Tg (No. ab96518, manufactured by Abcam) was added to 0 µg/mL (negative control), 1.0 µg/mL, or 10 µg/mL, to prepare model samples. To each model sample, anti-Tg antibody 64-1 was added to 50 µg/mL, to prepare an autoantibody-positive model sample. Tg-positive samples to which the anti-Tg antibody was not added were provided as autoantibody-negative model samples.

### (4) Sample Pretreatment

30 *µ* L of each sample was mixed with 50 µL of a pretreatment liquid (0.2 M NaOH). and the resulting mixture was left to stand at room temperature for 10 minutes. Subsequently, a neutralization liquid (0.2 M 11Cl, 50 µL) was added to the mixture. to carry out neutralization (pretreated sample). At the same time. 30 µL of each same sample was mixed with 50 µL of 0.2 M NaCl solution, and the resulting mixture was left to stand at room temperature for 10 minutes, followed by mixing with 50 µL of pure water (non-pretreated sample).

### (5) Tg Measurement

To each well of the anti-Tg antibody plate prepared in (1), 100 µL of a primary reaction liquid (0.02 M KH₂PO₄, 0.076 M K₂HPO₄, 0.25 M NaCl, 0.02 M EDTA 2Na, 1% PVP, 1% BSA, 0.05% casein Na, 0.05% Tween 20 (trade name; pH 7.3) was dispensed, and then 100 µL of the pretreated sample or the non-pretreated sample was added to each well. The plate was left to stand at room temperature for 60 minutes, and then washed with a washing liquid (0.05% Tween 20 (trade name), PBS) five times. To each well, 100 µL of the labeled antibody liquid was dispensed, and the plate was left to stand at room temperature for 30 minutes, followed by washing with a washing liquid (0.05% Tween 20 (trade name), PBS) five times. To each well, 100 µL of AMPPD substrate liquid (Lumipulse substrate liquid (manufactured by Fujirebio Inc.)) was dispensed, and luminescence was allowed to occur for 10 minutes while the amount of luminescence was measured at a wavelength of 477 nm using a microplate reader. The amount of luminescence (counts) under each condition is shown in Table 1.

**[Table 1]**

| Pretreatment | | No | | | Yes | | |
|---|---|---|---|---|---|---|---|
| Autoantibody | | Negative | Positive | Positive/ Negative | Negative | Positive | Positive/ Negative |
| Tg level (µg/mL) | 0 | 2810 | 3020 | 1.07 | 2715 | 2600 | 0.96 |
| | 1 | 37745 | 4775 | 0.13 | 9025 | 7965 | 0.88 |
| | 10 | 340775 | 20060 | 0.06 | 73770 | 58205 | 0.79 |

Based on comparison between the autoantibody-negative model samples and the autoantibody-positive model samples in the cases of detection of Tg under the condition without the pretreatment, the counts for the positive model samples were about one-tenth of the counts for the negative model samples, indicating that the signal remarkably decreases due to the influence of the autoantibody. On the other hand, under the condition with the pretreatment, while the overall count value decreased due to the influence of the alkali, the counts for the positive model samples recovered to about eight- to nine-tenths of the counts for the negative model samples, indicating that measurement of Tg is possible at a level almost equivalent to that in the case of a sample containing no autoantibody, even in the presence of the autoantibody in the sample.

### <Example 2 Test on Effects of Addition of Various Surfactants and Denaturants>

For the autoantibody-positive model samples in Example 1, measurement of Tg was carried out by the same method as in Example 1 except that a surfactant (a nonionic surfactant (Triton X-100 or Brij 35), a zwitterionic surfactant (CHAPS, C12APS, or C14APS), or an anionic surfactant (SDS or NLS)) or a denaturant (urea) was added to the pretreatment liquid such that the concentration during the pretreatment was as shown in Table 2. In the measurement under each condition, a sample pretreated with a pretreatment liquid containing neither a surfactant nor a denaturant was also subjected to the measurement. The measured counts under each condition are shown in Table 2.

**[Table 2-1]**

| Triton X-1 00 Concentration (%) | | 0 | 0.01 | 0.05 | 0.5 | 1 | 5 |
|---|---|---|---|---|---|---|---|
| Tg level (µg/mL) | 0 | 3190 | 3430 | 3355 | 3710 | 3435 | 4645 |
| | 1 | 5315 | 5420 | 8030 | 9315 | 9545 | 8095 |
| | 10 | 17525 | 43405 | 42575 | 42435 | 69465 | 57965 |

**[Table 2-2]**

| Brij 35 concentration (%) | | 0 | 0.01 | 0.05 | 0.5 | 1 | 5 |
|---|---|---|---|---|---|---|---|
| Tg level (µg/mL) | 0 | 2930 | 3160 | 3455 | 3285 | 3505 | 3145 |
| | 1 | 5905 | 6975 | 11265 | 9800 | 6620 | 11695 |
| | 10 | 29625 | 34205 | 88165 | 56900 | 64590 | 105875 |

**[Table 2-3]**

| CHAPS concentration (%) | | 0 | 0.01 | 0.05 | 0.5 | 1 | 5 |
|---|---|---|---|---|---|---|---|
| Tg level (µg/mL) | 0 | 3080 | 3245 | 3825 | 3330 | 4545 | 4280 |
| | 1 | 5825 | 6185 | 9015 | 7965 | 9365 | 6200 |
| | 10 | 13975 | 37035 | 41660 | 54505 | 55995 | 30095 |

**[Table 2-4]**

| C12APS concentration (%) | | 0 | 0.01 | 0.05 | 0.1 | 0.5 |
|---|---|---|---|---|---|---|
| Tg level (µg/mL) | 0 | 3080 | 2945 | 2945 | 2945 | 2815 |
| | 1 | 5825 | 5770 | 5670 | 5140 | 4250 |
| | 10 | 13975 | 26555 | 27775 | 28045 | 19995 |

**[Table 2-5]**

| C14APS concentration (%) | | 0 | 0.01 | 0.05 | 0.1 | 0.5 |
|---|---|---|---|---|---|---|
| Tg level (ng/_{mL}) | 0 | 3080 | 2760 | 2915 | 3080 | 2670 |
| | 1 | 5825 | 5670 | 5290 | 5355 | 6310 |
| | 10 | 13975 | 22005 | 18220 | 21955 | 18895 |

**[Table 2-6]**

| SDS concentration (%) | | 0 | 0.01 | 0.05 | 0.5 | 1 | 2.5 |
|---|---|---|---|---|---|---|---|
| Tg level (µg/mL) | 0 | 2600 | 3565 | 3405 | 2695 | 2915 | 3115 |
| | 1 | 7965 | 14615 | 14740 | 15955 | 9665 | 10075 |
| | 10 | 58205 | 100180 | 116115 | 176285 | 77660 | 57040 |

**[Table 2-7]**

| NLS concentration (%) | | 0 | 0.01 | 0.05 | 0.5 | 1 | 2.5 |
|---|---|---|---|---|---|---|---|
| Tg level (µg/mL) | 0 | 2600 | 2960 | 3040 | 2870 | 3040 | 3205 |
| | 1 | 7965 | 11200 | 14950 | 13360 | 18025 | 14795 |
| | 10 | 58205 | 86125 | 123110 | 183245 | 157790 | 128920 |

**[Table 2-8]**

| Urea concentration (M) | | 0 | 0.01 | 0.025 | 0.05 | 0.1 | 0.5 |
|---|---|---|---|---|---|---|---|
| Tg level (µg/mL) | 0 | 2370 | 2965 | 3200 | 3095 | 2440 | 2300 |
| | 1 | 7195 | 13570 | 12820 | 11265 | 11990 | 7965 |
| | 10 | 51025 | 97410 | 131565 | 100475 | 51605 | 77300 |

According to Table 2, it was shown that improvement of the signal of Tg can be achieved by carrying out both the alkali treatment and the addition of a nonionic surfactant, a zwitterionic surfactant, an anionic surfactant, or urea at an optimum concentration.

## Claims

1. A method of measuring, by an immunoassay, thyroglobulin in a sample separated from a living body, the method comprising a pretreatment step of mixing the sample separated from a living body with a pretreatment liquid containing an alkaline substance, wherein the pretreatment liquid further contains at least one selected from the group consisting of nonionic surfactants, zwitterionic surfactants, anionic surfactants, and urea.

2. The method according to claim 1, wherein the final concentration of the alkaline substance in the pretreatment step is more than 0.05 N and not more than 0.5 N.

3. Use of an alkaline substance for pretreatment in an immunoassay of thyroglobulin, wherein a pretreatment liquid for the pretreatment further contains at least one selected from the group consisting of nonionic surfactants, zwitterionic surfactants, anionic surfactants, and urea.

## Patentansprüche

1. Verfahren zum Messen von Thyreoglobulin in einer Probe, die von einem lebenden Körper abgetrennt wurde, mittels eines Immunassays, wobei das Verfahren einen Vorbehandlungsschritt des Vermischens der Probe, die von einem lebenden Körper abgetrennt wurde, mit einer Vorbehandlungsflüssigkeit umfasst, die eine alkalische Substanz enthält, wobei der Vorbehandlungsschritt weiters zumindest eines, ausgewählt aus der aus nichtionischen Tensiden, zwitterionischen Tensiden, anionischen Tensiden und Harnstoff bestehenden Gruppe umfasst.

2. Verfahren nach Anspruch 1, wobei die Endkonzentration der alkalischen Substanz im Vorbehandlungsschritt mehr als 0,05 N und nicht mehr als 0,5 N beträgt.

3. Verwendung einer alkalischen Substanz zur Vorbehandlung in einem Immunassay auf Thyreoglobulin, wobei eine Vorbehandlungsflüssigkeit für die Vorbehandlung weiters zumindest eines ausgewählt aus der aus nichtionischen Tensiden, zwitterionischen Tensiden, anionischen Tensiden und Harnstoff bestehenden Gruppe umfasst.

## Revendications

1. Procédé de mesure, par l'intermédiaire d'un dosage immunologique, de la thyroglobuline dans un échantillon séparé d'un corps vivant, le procédé comprenant une étape de prétraitement consistant à mélanger l'échantillon séparé d'un corps vivant avec un liquide de prétraitement contenant une substance alcaline, dans lequel le liquide de prétraitement contient en outre au moins un choisi dans le groupe comprenant des tensioactifs non ioniques, des tensioactifs zwitterioniques, des tensioactifs anioniques et de l'urée.

2. Procédé selon la revendication 1, dans lequel la concentration finale de la substance alcaline dans l'étape de prétraitement est supérieure à 0,05 N et n'est pas supérieure à 0,5 N.

3. Utilisation d'une substance alcaline pour un prétraitement dans un dosage immunologique de la thyroglobuline, dans laquelle un liquide de prétraitement pour le prétraitement contient en outre au moins un choisi dans le groupe comprenant des tensioactifs non ioniques, des tensioactifs zwitterioniques, des tensioactifs anioniques et de l'urée.
